# EUROPEAN PATENT SPECIFICATION

(11) **EP 3 453 425 B1**
(45) Date of publication and mention of the grant of the patent: **05.08.2026**
(21) Application number: 17848205.5
(22) Date of filing: 02.08.2017
(51) Int. Cl.: A61N 1/40, A61N 1/32

(54) **DIATHERMY TREATMENT DEVICE**
DIATHERMIEBEHANDLUNGSVORRICHTUNG
DISPOSITIF DE TRAITEMENT DIATHERMIQUE

(30) Priority: 09.09.2016 ES 201631180
(43) Date of publication of application: 13.03.2019
(73) Proprietor: Indiba, S.A., 08192 Sant Quirze del Valles (Barcelona) (ES)
(72) Inventor: CALBET BENACH, Jose, 08034 Barcelona (ES); RAMI MURILLO, Xavier, 08032 Barcelona (ES)
(74) Representative: Durán-Corretjer, S.L.P.
(86) International application number: PCT/ES2017/070564
(87) International publication number: WO 2018/046784

(56) References cited:
- EP-A1- 2 208 506
- EP-A1- 3 081 256
- WO-A1-2015/086873
- ES-U- 1 055 419
- ES-U- 1 063 601
- GB-A- 1 485 170
- GB-A- 1 485 170
- GB-A- 718 439
- US-A- 4 016 886
- US-A- 5 249 575
- US-A- 5 983 141
- US-A1- 2005 021 088
- US-A1- 2006 212 077
- US-A1- 2012 065 714
- US-B2- 7 951 061
- MEDINA LEONEL E. ET AL: "Nerve excitation using an amplitude-modulated signal with kilohertz-frequency carrier and non-zero offset", JOURNAL OF NEUROENGINEERING AND REHABILITATION, vol. 13, no. 1, 12 July 2016 (2016-07-12), pages 1 - 11, XP093020027, Retrieved from the Internet <URL:http://link.springer.com/content/pdf/10.1186/s12984-016-0171-4.pdf> DOI: 10.1186/s12984-016-0171-4
- JOHNSON MARK I. ET AL: "Characterising the Features of 381 Clinical Studies Evaluating Transcutaneous Electrical Nerve Stimulation (TENS) for Pain Relief: A Secondary Analysis of the Meta-TENS Study to Improve Future Research", MEDICINA, vol. 58, no. 6, 14 June 2022 (2022-06-14), pages 803, XP093020419, DOI: 10.3390/medicina58060803

## Description

The present description relates to diathermy treatment devices, that is to say treatment by means of applying electric current with the aim of causing an increase in temperature on parts of the human body. Said currents can be used to treat pain, for rehabilitation, traumatology, dermatology and sports medicine, and also in the specialty of cosmetics and cosmetic medicine. Diathermy is sometimes called hyperthermia therapy, although the term diathermy is preferred for distinguishing this type of treatment.

One type of known diathermy device comprises a generator and at least one neutral or return electrode and one active application electrode. The active electrode tends be smaller than the return electrode. In order to produce the diathermic effect, the generator is configured to produce alternating electricity at a fixed or adjustable frequency of between 0.1 and 40 MHz. However, the safe range of diathermic application is between 100 kHz and 10 MHz. Generally, the diathermic current is generated in a sinusoidal manner. Generally, this current is applied with a constant amplitude of the sinusoidal function. It is also known to emit pulse trains, that is to say alternating current pulses having constant amplitude and interspersed with periods of no current being applied. The average value of transmitted power is given by the effective amplitude of the signal and the power, in turn, defines the amount of heat transmitted to the body and therefore the diathermic effect.

In the field of electric scalpels, electric current is used for cutting tissue and dissecting. Electric scalpels typically use alternating electric current at frequencies of from 0.5 to 1.7 MHz and wattages of from 100 to 300 W, using an electrode that ends in a point in order to concentrate the power. The heat produced cuts the tissue. In electric scalpels, modulation of the amplitude of the current is used in order to stimulate coagulation of the proteins. The coagulated proteins thus seal the blood vessels sectioned by the scalpel in order to prevent them from bleeding.

ES2464241 B1 discloses a d'Arsonval current generator which generates trains of damped waves. Said currents have an exclusively superficial effect and produce decongestive, decontracting, sterilising, analgesic and neurotrophic effects and can therefore be used to non-invasively treat anorectal conditions.

US4016886A discloses a diathermic device having alternating currents in the range of between 100 kHZ and 1 MHz, modulated with a frequency of 100 Hz with the aim of maintaining the tissue at a constant temperature. To heat internal tissue, penetrative electrodes (needles) are used.

EP2208506 A1 discloses an example of a radiofrequency hyperthermia oncothermia device for the treatment of intraluminar or intracavitary lesions, particularly cancer, consisting of a catheter with an electrode and RF-independent highly isolated temperature sensing, a counter-electrode and a radiofrequency source which provide a radiofrequency fields whose amplitude is modulated in audio range. As any oncothermia device, the aim is to produce instantaneous necrosis and/or a heat-shock respose in cells leading to cell death. This aim is also shared by US7951061 B2, said document applying however an alternating magnetic field for heating, same alternating magnetic field being disclosed in US2005021088.

With respect to this known prior art, the present description pertains to a diathermy device comprising a generator and at least one neutral or return electrode and one active application electrode, the generator being configured to generate an alternating electric current having a frequency of between 100 kHz and 10 MHz, and characterised in that the generator is configured to modulate the amplitude of the alternating electric current using a modulation frequency of between 18 kHz and 30 kHz.

The applicant has found that, within an amplitude modulation in this range of frequencies (both of the alternating current and of the amplitude modulation), a compacting effect or an increase in density of the internal tissue is produced.

This novel effect has interesting applications:
- Therapeutic applications, since the compacting of internal tissue helps to stop small internal bleeds by means of pressure and, as a result, the applicant has found that recovery times are reduced.
- Cosmetic applications, since by compacting the tissue underneath the skin, a more satisfactory exterior cosmetic outcome is achieved than with solely superficial techniques.

Preferably, the modulation is also sinusoidal.

The modulation index (that is to say the quotient of the maximum amplitude and the minimum amplitude of the modulating wave) may be between more than 0% and less than 100%. The optimum modulation index may depend on the tissue to be treated and on the specific application. However, low values have a limited modulatory effect and high values result in an excessive loss of power with respect to the unmodulated wave and, as a result, a loss of the diathermic effect.

Therefore, the amplitude modulation will preferably have a modulation index of between 10% and 90%.

The applicant has found that, for general purposes, modulation indices of between 30% and 50% are particularly suitable.

For a better understanding, explanatory yet non-limiting drawings of an embodiment of the aim of the present teaching are appended by way of example, in which:
Fig. 1 is a graph showing alternating current of the type used in diathermy;
Fig. 2 is a graph showing alternating current having modulated amplitude obtained according to the present teaching;
Fig. 3 is a graph showing another alternating current having modulated amplitude obtained according to the present teaching.

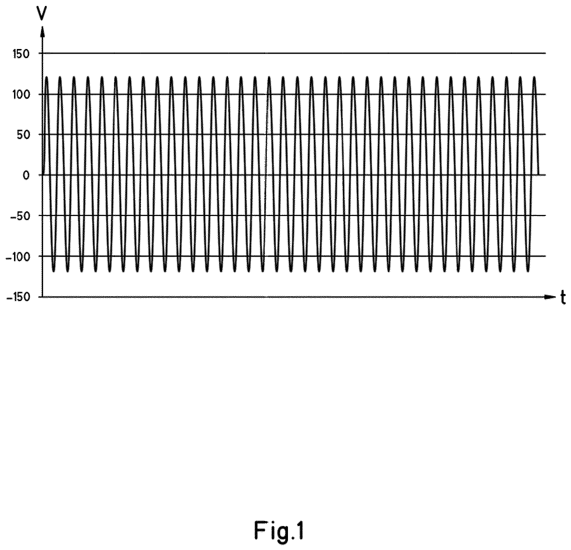

Fig. 1 shows voltage against time of a diathermic current that is obtained by means of a diathermic device of the known type. In particular, the generator of diathermic currents generates an alternating electric current that is sinusoidal and has a frequency of between 0.1 MHz and 10 MHz. As can be seen, the signal in this case is continuous (there are no pulse trains) and the amplitude of the current (that is to say the maximum voltage value in each cycle) is constant.

The present teaching equips a generator of the known type with amplitude modulation means (in this case, the voltage) of the alternating electric current, or alternatively the invention configures the existing means so as to obtain the desired current. Since the modulation of the amplitude of electric signals is an extensively known technical field, it is not necessary to explain said means in detail.

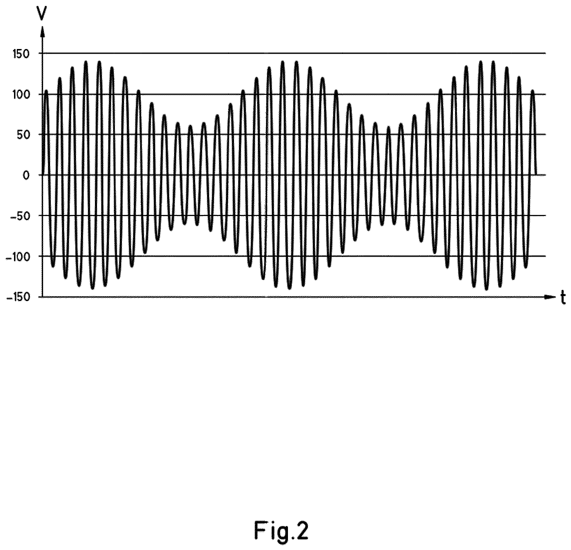

Fig. 2 shows a first example of diathermic electric current according to the present teaching. As can be seen, the current has the same frequency as the signal in Fig. 1. However, the amplitude is modulated by means of a sinusoidal signal or wave. The wave in question is called a modulating wave. The maximum values of amplitude conform to a sinusoidal shape having a frequency of between 18 and 30 kHz.

As can be seen, the modulation index, that is to say the relationship between the maximum and minimum value of amplitude of the modulating wave, is approximately 40%.

In this example, the modulation is likewise continuous, that is to say there are no periods of unmodulated current.

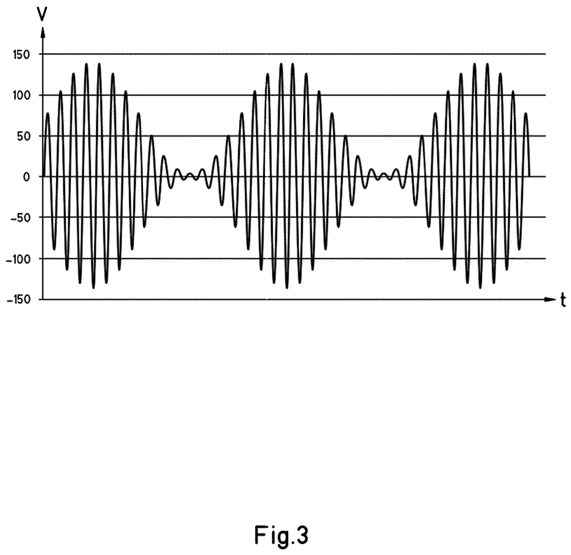

Fig. 3 shows a second example, similar to the previous, in which the modulation index is 100%. This embodiment, in principle, is not as advantageous because it involves a considerable decrease in the transmitted power and, therefore, a decrease in the diathermic effect. In order to compensate for the loss in diathermic effect (average power) with respect to the example in Fig. 1, it is necessary to increase the maximum voltage of the unmodulated electric current, which is undesirable due to risks of burns and/or electrocution.

The present invention is set out in the claims that follow.

## Claims

1. Diathermy treatment device comprising an alternating electric current generator and an electrode, the electrode and the generator being electrically connected and the generator being configured to generate alternating electric currents at a frequency within the range of from 100 kHz to 10 MHz, **characterised in that** the generator is configured to modulate the amplitude of the alternating electric current at a modulation frequency of between 18 kHz and 30 kHz, and **in that** the modulation has a modulation index of between 30% and 50%.

2. Device according to the preceding claim, **characterised in that** the modulation is sinusoidal.

3. Device according to either claim 1 or claim 2, **characterised in that** the modulation index is 40%.

## Patentansprüche

1. Diathermie-Behandlungsvorrichtung, umfassend einen Wechselstromgenerator und eine Elektrode, wobei die Elektrode und der Generator elektrisch miteinander verbunden sind und der Generator so ausgelegt ist, dass er Wechselströme mit einer Frequenz im Bereich von 100 kHz bis 10 MHz erzeugt, **dadurch gekennzeichnet, dass** der Generator so ausgelegt ist, dass er die Amplitude des Wechselstroms mit einer Modulationsfrequenz zwischen 18 kHz und 30 kHz moduliert, und dass die Modulation einen Modulationsindex zwischen 30 % und 50 % aufweist.

2. Vorrichtung nach dem vorstehenden Anspruch, **dadurch gekennzeichnet, dass** die Modulation sinusförmig ist.

3. Vorrichtung nach Anspruch 1 oder Anspruch 2, **dadurch gekennzeichnet, dass** der Modulationsindex 40 % beträgt.

## Revendications

1. Dispositif de traitement diathermique comprenant un générateur de courant électrique alternatif et une électrode, l'électrode et le générateur étant connectés électriquement et le générateur étant configuré pour générer des courants électriques alternatifs à une fréquence comprise dans une plage de 100 kHz à 10 MHz, **caractérisé en ce que** le générateur est configuré pour moduler l'amplitude du courant électrique alternatif avec une fréquence de modulation comprise entre 18 kHz et 30 kHz, et **en ce que** la modulation présente un indice de modulation compris entre 30 % et 50 %.

2. Dispositif selon la revendication précédente, **caractérisé en ce que** la modulation est sinusoïdale.

3. Dispositif selon la revendication 1 ou la revendication 2, **caractérisé en ce que** l'indice de modulation est de 40 %.
